# EUROPEAN PATENT APPLICATION

(11) **EP 4 841 645 A1**
(43) Date of publication of application: **18.11.2026**
(21) Application number: 26166319.9
(22) Date of filing: 20.03.2026
(51) Int. Cl.: G16H 50/20, G16H 40/60, G16H 50/50

(54) **INTEGRATED REMOTE BIOMEDICAL AND BIOMETRIC MONITORING SYSTEM WITH STRATIGRAPHIC, COGNITIVE AND ADAPTIVE ARTIFICIAL INTELLIGENCE, BASED ON COMPLETELY BIODEGRADABLE MYCELIAR RETICULAR NETWORKS**

(30) Priority: 14.05.2025 IT 202500010782
(71) Applicant: NIT - Naples Institute of Technologies di Luca Mercogliano, 80126 Napoli (NA) (IT)
(72) Inventor: Mercogliano, Luca, 80126 Napoli (IT)
(74) Representative: Garavelli, Paolo

(57) **Abstract**

The present invention discloses an integrated and modular system for the remote monitoring and evaluation of biomedical, biometric and clinical parameters, comprising a digital telematics platform (108, 110, 114, 116), a plurality of intelligent wearable devices (106) based on woven mesh polymers equipped with integrated micro-sensors, and a Big Data Platform with distributed artificial intelligence modules, intended for the analysis, inference, recommendation and personalization of diagnostic, therapeutic and rehabilitative services; the system includes a Patient-Centric Environment subsystem (106) for remote monitoring, biomedical databases, a fruition subsystem with personalized user interfaces, and an interaction subsystem for patients, doctors and healthcare professionals (120); the platform processes the collected data to provide functional assessments, rehabilitation planning, predictive diagnostics and continuous feedback.

## Description

The present invention relates to remote biomedical and biometric monitoring systems, and more specifically to an integrated and modular system for the remote monitoring and evaluation of biomedical (and biometric) and/or clinical parameters based on an artificial intelligence platform structured by multiple three-dimensional layers of different AI (Artificial Intelligence) codes and multiple microscopic reticular-type devices based on mycelial structures (different mycelia integrated with each other) in turn integrated into smart wearable yarns and fabrics (IoT).

Continuous monitoring of biometric and clinical parameters is playing an increasingly important role in managing people health, work, and psychophysical and athletic well-being, with the specific aim of personalising and improving the precision of treatments.

Recent advances in wearable technologies (IoT) and artificial intelligence (various models) have opened up new possibilities for collecting and analysing physiological data in real time.

These systems allow monitoring parameters such as heart rate, blood pressure, blood oxygen levels and many other indicators of an individual health.

However, currently available monitoring systems have some limitations.

Many wearable devices have very limited battery life, which limits their prolonged use. Furthermore, many of them cannot be washed thousands of times and are generally made of polluting materials anyway.

Furthermore, the accuracy and reliability of measurements can be compromised by factors such as movement or incorrect positioning of the sensors which lead to a strong "burst" and different background noise (high noise levels).

Another major criticality concerns the integration of collected signals and the interpretation of data from different sources, which requires extremely advanced algorithms to extract clinically relevant information (lack of precision, accuracy, repeatability, reproducibility, efficiency, efficacy, sensitivity, specificity and reliability in "data mining").

Personalizing monitoring, aimed at precision care, based on the specific needs of each patient represents an additional challenge. Current systems often use standardized thresholds and parameters (on small experimental samples) that do not account for individual variability (the uniqueness of the subject being examined).

Furthermore, managing large amounts of sensitive health data securely and in compliance with privacy regulations raises cybersecurity and personal information protection concerns.

It has been understood that a system is needed that overcomes () several of these problems simultaneously.

The present invention relates to remote biomedical (and biometric) monitoring systems, and more specifically to an integrated and modular system structured on multiple levels (3D) for the remote monitoring and evaluation of all clinical parameters that can be collected on a patient based on an artificial intelligence platform and intelligent wearable devices.

The above and other objects and advantages of the invention, as will result from the following description, are achieved with an integrated biomedical and biometric monitoring system such as that described in claim 1. Preferred embodiments and non-trivial variants of the present invention form the subject of the dependent claims.

It is understood that all attached claims form an integral part of this specification.

In a first aspect, an integrated and modular system for the remote monitoring and evaluation of biometric and clinical parameters is provided.

The system comprises a digital telematics platform, a plurality of intelligent wearable devices based on the integration of various woven polymers, carefully matched to specific mycelial networks based on micro- and nano-grids that form the BUS, a complex set of common intelligent communication lines that allow the components (modules, devices, and/or equipment) of the electronic system to easily communicate with each other by exchanging binary data. The networks are also equipped with micro-sensors integrated into the intelligent mycelial networks (of different types of mycelium) and connected to the Big Data Platform module, equipped with various distributed artificial intelligence modules. This platform features multiple layers of different artificial intelligence techniques and code methods (SCAAI design) for data collection, analysis, inference, machine learning and recommendation, and discretization to best prepare the personalized delivery of diagnostic, therapeutic, medical-rehabilitative, and/or work and sports services.

This integrated system enables continuous and non-invasive monitoring of patient biometric parameters, providing real-time data and advanced analytics to support personalized clinical decisions. The use of intelligent polymer fabrics improves patient comfort and adherence, while layered and distributed artificial intelligence-including within the BUS via mycelium-enables sophisticated data processing to generate meaningful clinical insights.

The system may include wearable devices including, but not limited to: T-shirts, pants, underwear, gowns, hoods, smart insoles for continuous sensing of physiological signals.

This variety of wearable devices allows for monitoring a wide range of biometric parameters from different parts of the body, providing a complete, real-time picture of the patient health status and any progress made in the treatment plan established during therapeutic planning. The diversification of detection points increases the overall accuracy of monitoring and reduces the risk of false positives or negatives. However, the most important aspect is that the main communication channel located in the mycelial BUS allows the peripherals and components of the entire electronic system spread across the wires, fabrics, and meshes to interface with each other, exchanging various types of information or data through the transmission and reception of all signals and all SCAAI (Stratify Cognitive Adaptive Artificial Intelligence) extrapolations.

At the same time, yarns, fabrics, and lattice structures can have a hexagonal geometry with AI integrated into the vertices and in turn form small modules, small devices if coupled together, small systems if combined with other modules, and if paired together, act as portions of even more complex systems.

This specific geometric structure optimizes the distribution of sensors and processing nodes, improving the sensitivity and energy efficiency of the system.

Integrating AI directly into the facility core capabilities enables on-site data pre-processing, reducing network load and improving system responsiveness.

The Big Data Platform can be composed of the modules: Data Fusion, Semantic Knowledge, Rule Engine, Recommender System, Machine Learning and Neural AI Engine.

This modular Big Data Platform architecture enables multidimensional analysis of biometric data, combining different artificial intelligence techniques to extract maximum information value.

The presence of specialized modules allows the analysis to be adapted to specific clinical and research needs, improving the diagnostic and predictive accuracy of the system.

User interfaces can include web portals, mobile apps, smart glasses, headsets, and customized dashboards.

Diversification of user interfaces increases the accessibility and usability of the system for different categories of users, from patients to healthcare professionals.

Customized dashboards and advanced visualization tools facilitate quick and intuitive interpretation of complex data, improving the effectiveness of clinical decision-making.

Data can be stored both locally and in cloud and analysed using semantic inference and neural networks appropriately anchored in the SCAAI layers.

This hybrid storage strategy ensures continuous data availability, even in the event of connectivity issues, and enables distributed processing that balances privacy needs with computational power.

Using advanced techniques such as semantic inference and neural networks allows us to extract complex patterns and hidden relationships from data, enhancing the system diagnostic and predictive capabilities.

It will be immediately obvious that countless variations and modifications can be made to what is described (for example relating to shape, dimensions, arrangements and parts with equivalent functionality) without departing from the scope of the invention as appears from the appended claims.

The present invention will be better described by some preferred embodiments, provided by way of example and not by way of limitation, with reference to the attached drawings, in which:

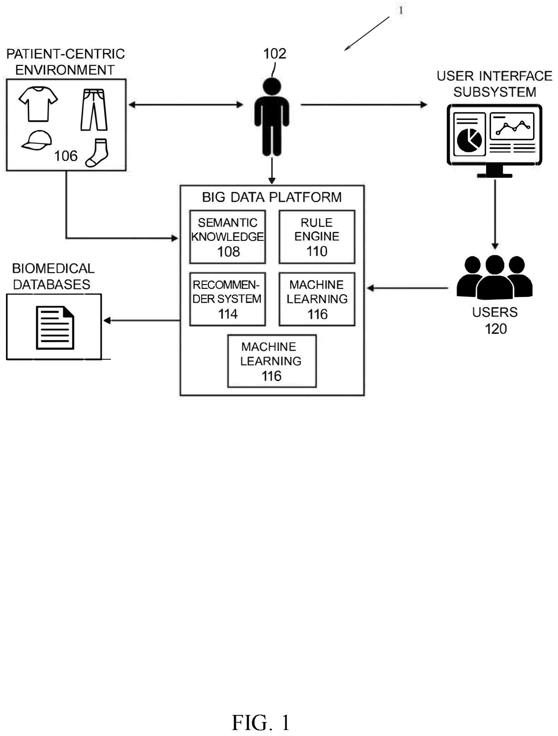

- FIG. 1 shows the basic modes of operation of a preferred embodiment of the operation/use model according to the present invention;

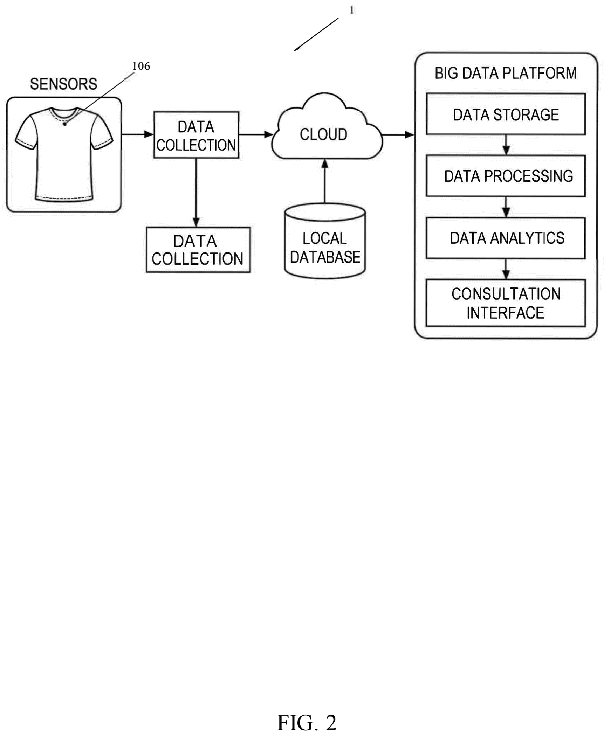

- FIG. 2 shows the "data collection" criteria for the data collected from the garments and the local and cloud repository methods for analysis, storage and final interaction;
- FIG. 3 represents a non-exhaustive example of which and how many wearables can contain the present invention;
- FIG. 4 is a generalization and an example of the complexity of sensor devices that can be embedded in clothing in order to illustrate that their relative arrangement can facilitate different biomedical and biometric measurements;
- FIG. 5 shows the block diagram of operation and interaction between modules, signals, interpretation and recommendation contained in the present invention;

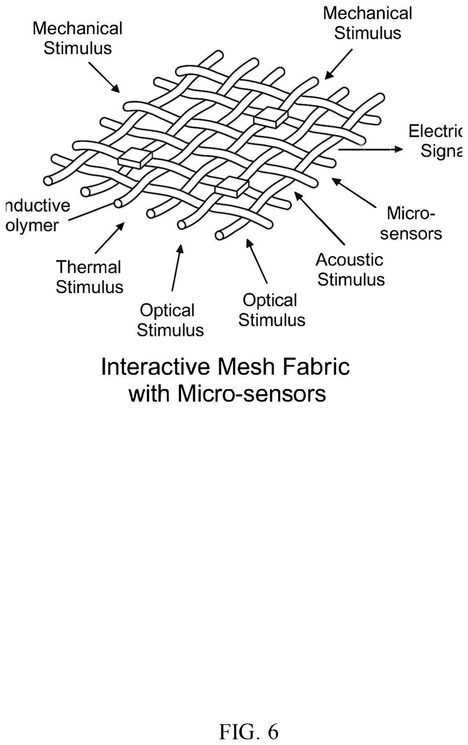

- FIG. 6 shows in a non-exhaustive way one of the possible inter-weavings that characterise the mycelial networks and at the vertices of the inter-weavings the flexible and stretchable electronic components in which the micro and nano electronic components and the nano and micro sensors are located;

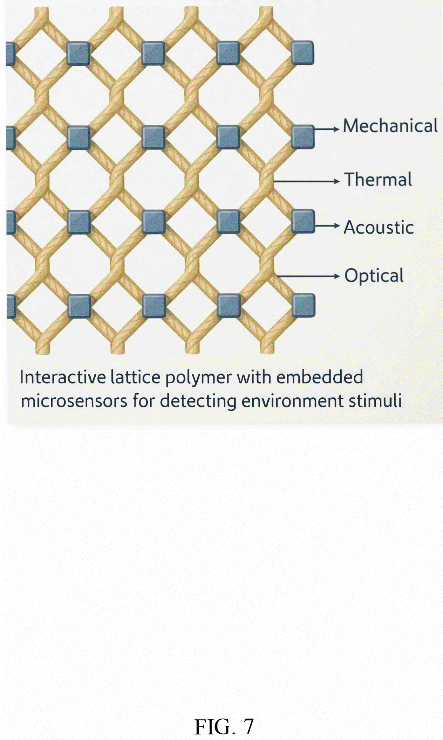

- FIG. 7 highlights, by way of example and not exhaustively, the different techniques and technologies that characterise the different signals that can be collected from the flexible and stretchable electronic component anchored to the vertices of the mycelial networks, better identifying the detection and tracking systems of the biomedical and physiological stimuli detectable on the body;

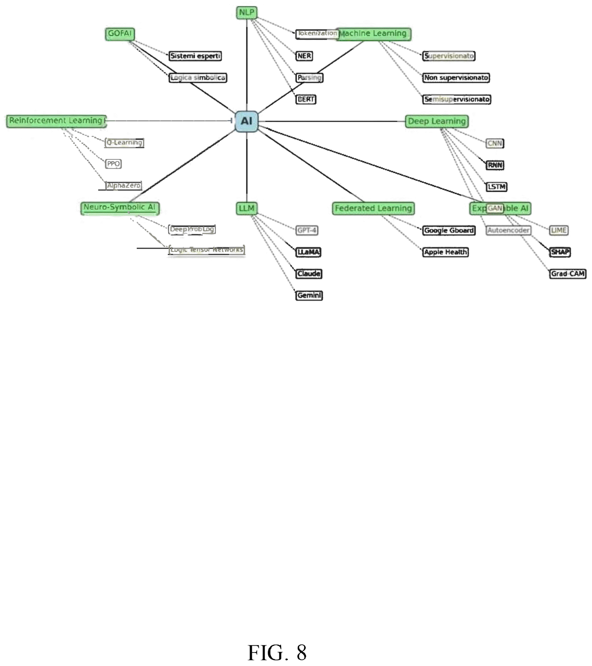

- FIG. 8 represents an example of the conceptual map used to write the source code of the SCAAI Artificial Intelligence designed for this invention;

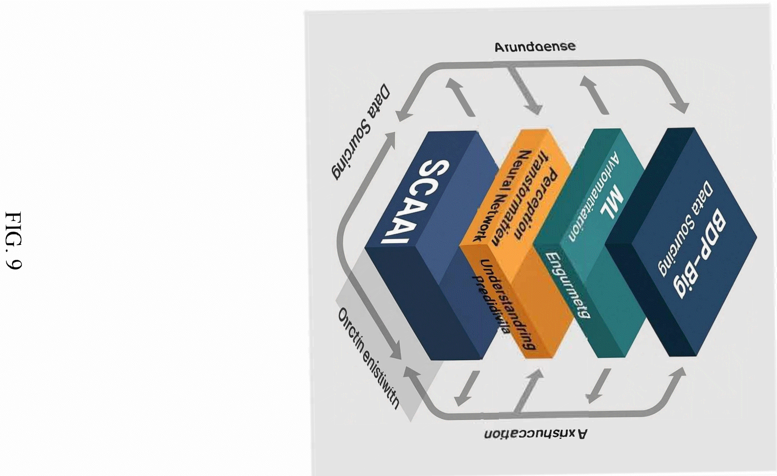

- FIG. 9 shows in an exemplary and non-exhaustive way one of the three-dimensional modalities of SCAAI layers that are representatively embedded in the stimulus or biomedical signal detection modules and that identify the 3D interaction modalities of the different AI techniques;

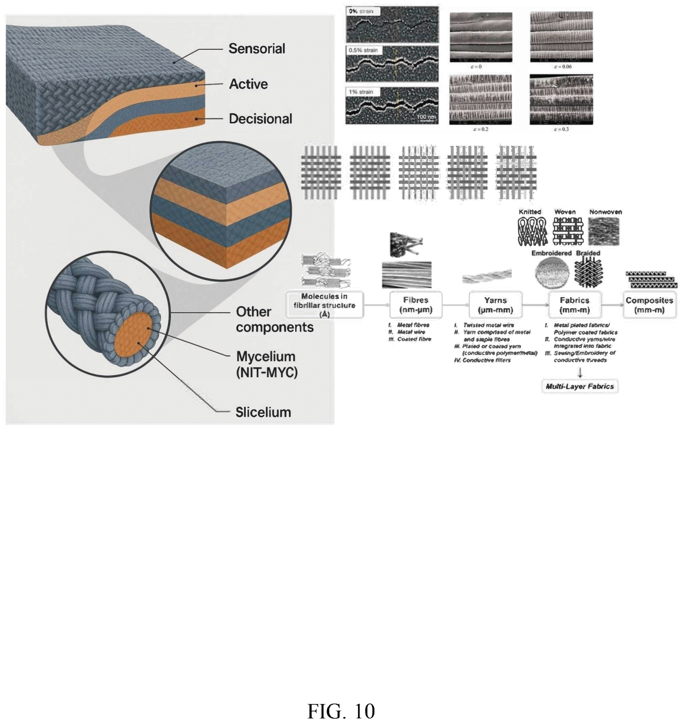

- FIG. 10 shows a non-exhaustive example of how fungal networks can be coupled by drowning them in the yarn and how they can then become essential constituents of the final fabric;

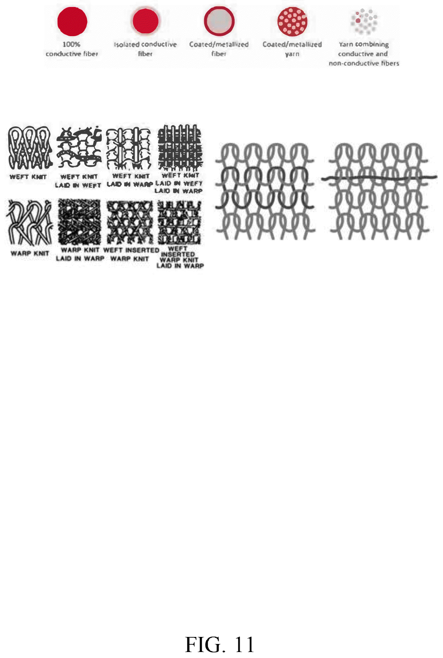

- FIG. 11 shows only a very few of the solutions that can be undertaken in the manufacture of multifunctional yarns and extremely sophisticated fabrics characterized by different levels of functionality present in this invention;

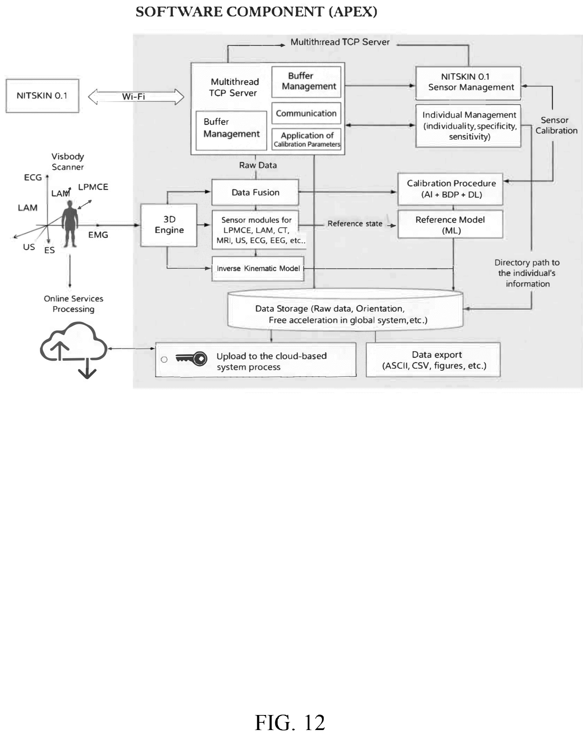

- FIG. 12 shows a reduced version of the complexity of the project constituting the APEX software component and illustrates with a block diagram the interactions of the various IT factors;

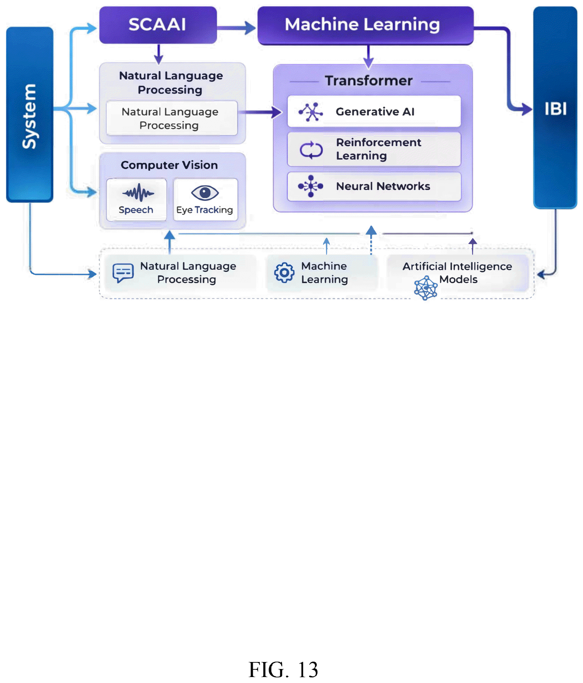

- FIG. 13 shows one of the typical examples illustrating how, for example, the multiple layers (22 layers on x, 22 layers on y and 22 layers on z) of coupled artificial intelligences present in the present invention can be made to interact;

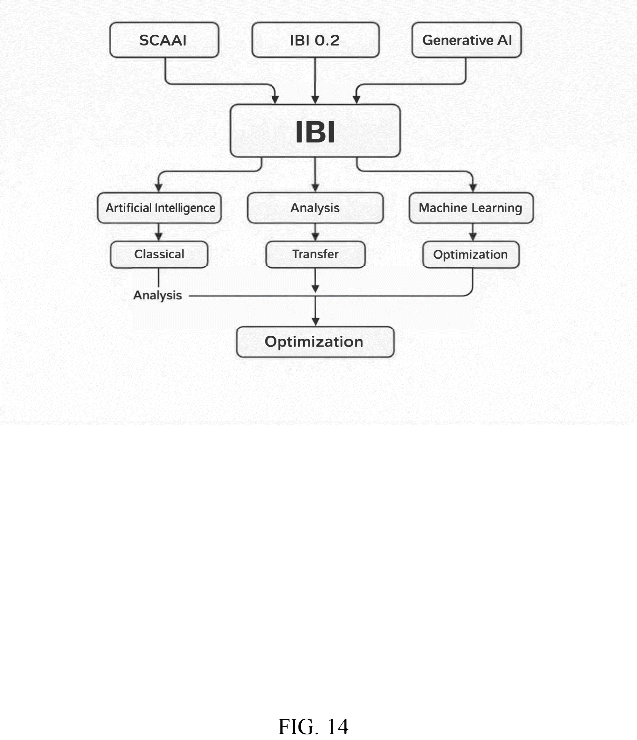

- Fig. 14 highlights one of the possible information flow optimization activities that identify, intercept, analyse, understand, compare, interpret the collected signals and suggest appropriate recommendations.

The integrated remote biometric monitoring system represents an innovative solution for monitoring and evaluating physiological parameters in the healthcare, sports, and work environments.

The platform is characterized by its layered, lattice-like, and modular nanoscale architecture, which combines intelligent wearable devices with an AI-based software infrastructure that is also layered and synergistic-modular.

This systemic integration between HW and SW allows for the collection, analysis and processing of a wide range of biometric data in real time, providing detailed and personalized information on the health status and performance of users.

The use of advanced artificial intelligence technologies in various technological forms allows for the sophisticated interception and interpretation of collected data, generating clinically relevant insights and supporting the medical and therapeutic decision-making process.

The modular nature of the system and the underlying mycelial structures guarantee its flexibility and adaptability to different needs and application contexts, allowing for the customization of the services offered based on the specific needs of users and healthcare professionals.

The final architecture, which adopts distributed techniques and technologies, also facilitates integration with existing systems and future expandability of the platform.

The Patient-Centric Environment (PCE) subsystem comprises a centralized digital environment for remote patient monitoring. The PCE uses a series of 106 innovative wearable devices made from specialized mycelial yarns, fabrics with integrated smart polymers, and various interactive multilayered fabric meshes equipped with integrated microsensors to detect environmental stimuli and convert them into electrical signals.

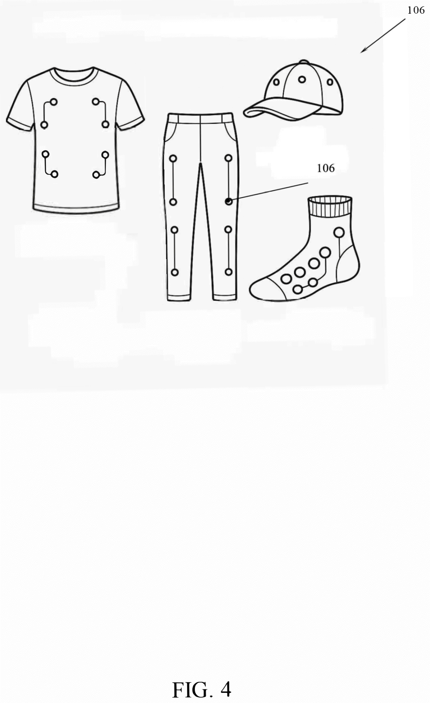

As illustrated in FIG. 4, the wearable device series 106 includes different types of smart garments and accessories 107, 109, 110:
- medical shirts and gowns for electrocardiogram (ECG), body temperature, and blood pressure monitoring. These garments feature textile sensors integrated into the fabric for non-invasive and continuous measurements;
- pants equipped with electromyography (EMG) sensors. The sensors are strategically placed to detect muscle activity in the lower limbs;
- electroencephalography (EEG) caps. These caps contain dry electrodes embedded in the tissue for monitoring brain activity;
- smart socks and insoles for plantar pressure sensing. These devices use distributed pressure sensors to analyse weight distribution and foot biomechanics;
- wearable devices for 3D motion analysis, such as bracelets or elastic bands, which integrate accelerometers and gyroscopes to track body movements;
- facial fatigue detection systems, such as smart glasses or headbands, equipped with optical sensors to monitor eye movements and facial expressions.

The 106 series of wearable devices are equipped with IoT sensors that use the MQTT protocol for data transmission. Additionally, the devices support other protocols suitable for NFC i-Bicon data transfer, ensuring efficient and secure communication with the central system.

The mesh polymer fabrics used in wearable devices feature an innovative structure that allows for the integration of active and passive microsensors. This configuration allows for accurate and continuous biometric data collection while maintaining user comfort and convenience.

The Big Data Platform (BDP) is an advanced system for managing, analysing, and inferring biometric and clinical data. The BDP is composed of several interconnected modules, each with specific capabilities for processing data from wearable devices and other sources.

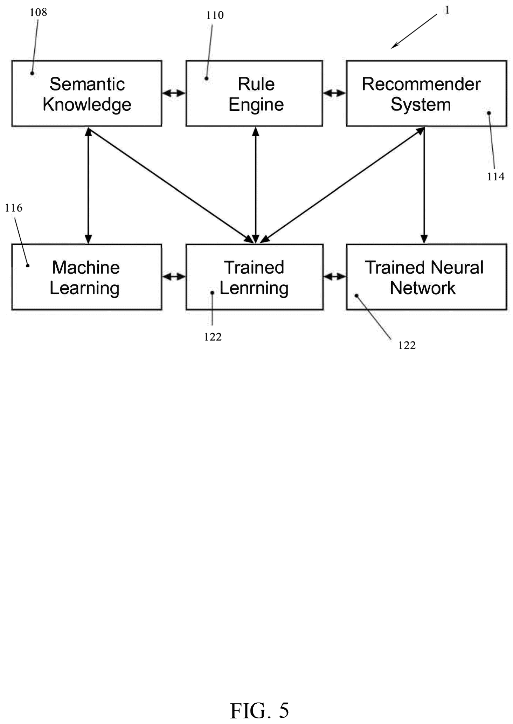

As illustrated in FIG. 5, the BDP includes a semantic knowledge module 108, a data interface 110, a recommender module 114, and a machine learning module 116.

A semantic knowledge module 108 uses an AI-enhanced neural network to generate semantic graphs. These graphs are useful for supporting diagnosis and treatment planning. For example, the module can analyse a patient electrocardiogram data and correlate it with historical clinical information to suggest potential cardiac diagnoses.

A data interface 110 integrates heterogeneous biometric and clinical data streams from various sources. This module normalizes and harmonizes data into a consistent format for subsequent analysis.

A recommender module 114 implements a personalized recommendation system. Based on patient data and predictive models, this module can suggest therapeutic interventions or changes to the treatment plan. For example, it might recommend a drug dose adjustment based on continuous monitoring of vital signs.

A machine learning module 116 includes algorithms for data classification, regression, and prediction. This module can be used, for example, to predict the risk of postoperative complications based on preoperative data and the initial postoperative course.

The BDP also includes a Neural AI Engine with deep neural networks for predictive modelling of patient data. This engine can analyse time series of biometric data to identify patterns and trends that aren't immediately obvious, thus supporting early diagnosis and prevention.

All these modules work synergistically, communicating through an interoperable, modular architecture. The processed data is stored both locally and in cloud, allowing for flexible management of time series and associated metadata.

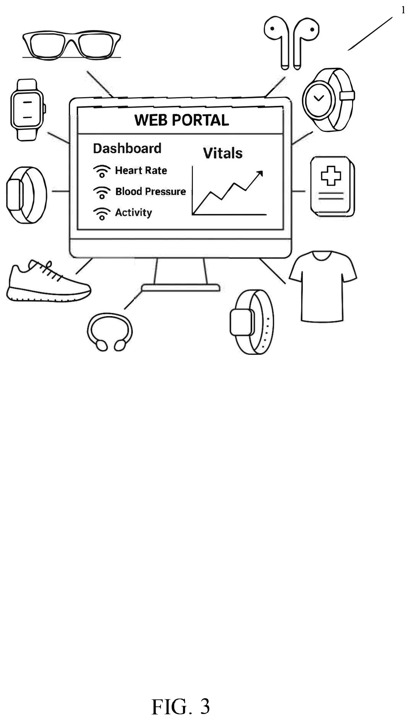

The fruition subsystem includes various user interfaces and tools for accessing the personalized services offered by the platform, as illustrated in FIG. 3.

A web portal with dynamic dashboards allows users to view and interact with data in real time. Dashboards can be customized to display metrics and information relevant to each user, such as vital signs graphs, rehabilitation progress, or alerts on potential health issues.

Mobile and tablet applications offer access to the platform's services from portable devices. These apps may include features such as medication reminders, symptom tracking, or direct communication with medical staff.

For a more immersive experience of the data, the system integrates augmented and virtual reality devices such as smart glasses, earphones, and headphones. These allow, for example, viewing 3D models of the patient body superimposed on reality, or accessing detailed instructions for rehabilitation exercises.

The subsystem also includes an automated and constantly updated Electronic Health Record. This collects all the patient clinical data in a structured manner, allowing for a holistic view of their medical history.

All these interfaces are designed to present information clearly and intuitively, adapting to the needs and preferences of each user.

For example, a doctor might see detailed graphs of vital signs trends, while a patient might see a simplified version with easy-to-understand indicators.

The Users and Interaction subsystem includes different types of users who interact with the system in a personalized manner; system 120 users include patients, doctors, and healthcare professionals, each with different access profiles.

For patients, the system provides a simplified interface that allows them to view their biometric data, receive feedback on their health status, and follow treatment recommendations.

A representation of patient 102 is created within the system to store and analyse data specific to each individual.

Clinicians have access to advanced features for diagnosis, monitoring, and treatment planning; they can view detailed data, historical trends, and predictions generated by the system artificial intelligence modules.

Healthcare providers have a dedicated interface for operational management, such as scheduling appointments and monitoring patient adherence to prescribed therapies.

The system dynamically adapts the information and features displayed based on the logged-in user profile, ensuring that each user category has access only to the data and actions appropriate to their role. This modular approach allows for personalized and secure interaction with the system.

The integrated system described in the invention is based on the synergistic interaction between different subsystems to provide complete remote monitoring and evaluation of biometric and clinical parameters.

Referring to FIG. 5, it is possible to observe the logical connections between the various distributed artificial intelligence modules that make up the platform.

The data flow begins with a representation of the patient 102, which represents the primary source of biometric information. This information is collected through a wearable array 106, which includes sensors embedded in the smart mesh polymer fabrics; the collected data is then transmitted to a data interface 110, which serves as the input point for the processing system.

Once acquired, the data is processed by a semantic knowledge module 108. This module uses advanced artificial intelligence techniques to interpret the incoming data, generating semantic graphs that represent the relationships between the various biometric and clinical parameters. This semantic processing allows for a deeper understanding of the patient health status.

In parallel, a machine learning module 116 analyses the data to identify patterns and trends. This module applies classification, regression, and prediction algorithms to extract meaningful insights from the raw data.

The information processed by the semantic knowledge module and the machine learning module is then combined to provide a comprehensive and detailed view of the patient status.

Based on this integrated analysis, a recommendation module 114 generates personalized suggestions for patient management. These may include proposed therapeutic interventions, modifications to the treatment plan, or early warnings based on trends detected in the data.

Users of the 120 system, which may include physicians, healthcare professionals, and patients themselves, interact with the platform through various interfaces, as described above. These interfaces present the processed information and recommendations in a clear and accessible manner, enabling informed and timely decisions.

The continuous interaction between these subsystems creates a feedback loop in which data is constantly collected, analysed, and used to improve the accuracy of forecasts and recommendations. This dynamic and adaptive approach enables increasingly accurate customization of the services offered, improving the effectiveness of remote monitoring and evaluation.

## Claims

1. Integrated system for remote biometric monitoring, comprising:
- a plurality of wearable devices (106) equipped with integrated biometric sensors;
- at least one modular data analysis platform (108, 110, 114, 116) configured to process biometric data acquired from wearable devices (106), the platform comprising:
- at least one semantic knowledge module (108) to generate semantic graphs based on biometric data;
- at least one data interface (110) for integrating heterogeneous data streams;
- at least one recommendation module (114) to generate personalized suggestions;
- at least one machine learning module (116) for predictive data analytics; and
- at least one user interface configured to present the analysed data and recommendations to users of the system (120).

2. System according to claim 1, wherein the wearable devices (106) comprise at least one of: shirts, pants, hoods, socks, insoles and smart glasses.

3. System according to any of the preceding claims, wherein the wearable devices (106) are made of mesh polymer fabrics equipped with integrated micro-sensors.

4. System according to any of the preceding claims, wherein the user interfaces comprise at least one of: a web portal with dynamic dashboards, mobile applications, augmented and virtual reality devices.

5. Method for monitoring and analysing biometric parameters using the system of claim 1, comprising the steps of:
- acquiring biometric data from wearable devices (106);
- processing the acquired data using the modular data analysis platform (108, 110, 114, 116), including: generate semantic graphs based on biometric data using the semantic knowledge module (108);
- integrating heterogeneous data streams through the data interface (110);
- generating personalized suggestions using the recommendation module (114);
- performing predictive analytics on data using the machine learning module (116);
- presenting the analysed data and recommendations to system users (120) through user interfaces.

6. Method according to claim 5, further comprising the step of storing the processed data both locally and in the cloud.

7. Method according to claim 5 or 6, wherein the data processing comprises the application of semantic inference techniques and neural networks.

8. Software product comprising instructions that, when executed by a processor, implement the modular data analysis platform (108, 110, 114, 116) of the system of claim 1, the instructions configured to:
- receive biometric data from wearable devices (106);
- generate semantic graphs based on biometric data using the semantic knowledge module (108);
- integrate heterogeneous data streams through the data interface (110);
- generate personalized suggestions using the recommendation module (114);
- perform predictive analytics on the data using the machine learning module (116); and
- provide the analysed data and recommendations to the user interfaces for presentation to system users (120).

9. Software product according to claim 8, wherein the instructions are further configured to dynamically adapt the information and functionality displayed based on the profile of the logged in user.

10. Software product according to claim 8 or 9, wherein the instructions are further configured to automatically generate and update an electronic health record for each representation of the patient (102).
